# EUROPEAN PATENT SPECIFICATION

(11) **EP 3 801 451 B1**
(45) Date of publication and mention of the grant of the patent: **28.01.2026**
(21) Application number: 19724510.3
(22) Date of filing: 20.05.2019
(51) Int. Cl.: A61K 8/49, A61K 8/55, A61Q 19/02, A61K 8/06

(54) **NOVEL USE OF PROLINE DERIVATIVES**
NEUARTIGE VERWENDUNG VON PROLINDERIVATEN
NOUVELLE UTILISATION DE DÉRIVÉS DE PROLINE

(30) Priority: 24.05.2018 EP 18174098
(43) Date of publication of application: 14.04.2021
(73) Proprietor: DSM IP Assets B.V., 6221 BE Maastricht (NL)
(72) Inventor: CAMPICHE, Remo, 4303 Kaiseraugst (CH)
(74) Representative: dsm-firmenich IP
(86) International application number: PCT/EP2019/062993
(87) International publication number: WO 2019/224158

(56) References cited:
- FR-A1- 3 029 108
- KR-A- 20150 011 726
- US-A1- 2009 111 731
- US-A1- 2011 033 507
- US-A1- 2017 119 646
- US-A1- 2017 209 350
- US-A1- 2018 042 840

## Description

The present invention relates to a novel use of proline containing dipeptides for evening out as well as brightening the skin tone. These proline containing dipeptides are furthermore particular suitable for improving the skin tone of the cheek under the eyes, on the cheek and the jawline.

Healthy skin is generally characterized by an even, uniform coloration of the skin. Human skin, however, often exhibits small areas affected by adverse skin conditions associated with redness, inflammation, discoloration as well as hyperpigmentation. For example, rosacea, a common and chronic disorder, is characterized by flushing and persistent redness (erythema) in the central facial area. Such skin conditions, in particular when occurring in the face, may have considerable psychosocial impact on an individual and can cause embarrassment, anxiety and low self-esteem.

Although there are medications that are approved for the treatment of skin inflammation and/or redness respectively the treatment of hyperpigmented skin, there exists a need for mild agents which reduce the appearance of uneven skin tone and provide an even, uniform coloration of the skin.

US 2011/033507 discloses the use of polypeptides, that can include a proline, for the treatment of reducing dark circles under the eyes.

US 2017/119646 discloses a method of treating ageing related skin changes, especially hyperpigmentation of skin, age spots, erythema, uneven skin tone, by topically administering to skin a dipeptide derivative that can include a proline.

US 2018/0042840 discloses the use of a ferment extract to treat skin, especially depigmentation, whitening, or lightening in colour of the skin especially of dark eye circles. The composition can comprise an additional anti-wrinkle and/or anti-ageing agent such as SYN^{®}-AKE.

US 2017/0209350 discloses the use of dicarboxylic acids to treat skin, especially to reduce the production of melanin in the skin, to lighten the skin and its appendages, to attenuate skin spots, to homogenize skin colour. The composition can comprise an additional anti-wrinkle and/or anti-ageing agent such as SYN^{®}-AKE.

Surprisingly, it has been found that certain proline containing dipeptides are capable of evening out the skin tone thus resulting in a more even, homogeneous skin coloration. Thus, the first object of the present invention relates to the use of a compound of formula (I) 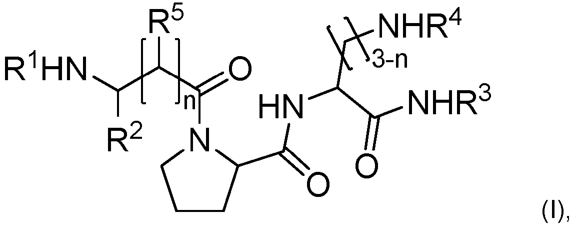 wherein
n represents 0, 1 or 2,
R¹ and R⁴ - independently of each other - are selected from the group consisting of
H, C₁-C₆alkyl, amidino or tetra-C₁-C₆-alkylamidinium,
R² is H or C₁-C₆-alkyl, or R¹ and R² together with the residue to which they are bound form a 5- to 7-membered, saturated ring;
R³ is selected from the group consisting of C₁-C₆alkyl, arC₁-C₆alkyl and heteroarylC₁-C₆alkyl; and
R⁵ is H and, when n is 1, also NH₂, or R⁵ and R¹ together with the residue to which
they are bound form a 5- to 7-membered, saturated ring;
or a cosmetically acceptable salt thereof for evening out skin tone, for brightening of skin and/ or for reducing dark circles under the eye area.

The present invention also relates to the subject-matter as further claimed.

The second object of the present invention relates to a compound of formula (I), as defined above, or a cosmetically acceptable salt thereof for use in the treatment of reducing inflammation-related discoloration, erythema and/or pigmentation of skin.

The phrase "cosmetically acceptable," as used herein is intended to mean that the particular component is regarded as safe and non-toxic for application to a human such as in particular to human skin at the levels employed.

Without wishing to be bound by theory it is believed that the evening out of the skin tone with the compound of formula (I) is achieved (at least inter alia) by removing the redness as well as the little dark spots under the skin (also called hidden pigmentation), which also results in a brighter (lighter) skin tone.

The evening out of the skin tone respectively the reduction of redness and/ or the brightening of the skin can be determined using the CILAB color space, (also known as CIE L*a*b* or sometimes abbreviated as simply "Lab" color space) which expresses color as three numerical values: L* for the black-white (i.e. the lightness), a* for the green-red and b* for the blue-yellow color components. The evening out of the skin color resulting in a visible improvement of the skin tone is in particular reflected by an increase of the L* value (increase in the lightness) and a decrease of the a* value (reduction of redness) as illustrated in the examples.

The L*a*b* values can be determined either with a Chroma Meter directly on the skin or by using photos taken under standardized methods using a mathematical algorithm to calculate the L*a*b* values of certain areas of the skin in order to determine the relative improvement versus non treated respectively placebo treated skin.

In a particular advantageous embodiment, the present invention relates to uses and methods according to the present invention wherein the L* value of the skin is increased and the a* value is decreased when compared to non-treated respectively placebo treated skin. Preferably, the L* value is increased by at least 0.1, preferably by at least 0.13 units and the a* value is decreased by at least 0.1, preferably by at least 0.13 units.

The uses and methods according to the present invention can further comprise the identification of a person in need of evening skin tone.

In a specific embodiment, the invention also relates to the use of a compound of formula (I) with all the preferences and definitions given herein to increase the L* value (i.e. the lightness) and decrease the a* value of skin treated with a compound of formula (I) compared to untreated skin and optionally appreciating the effect.

In another embodiment the invention relates to a method of reducing the appearance of uneven skin tone comprising topically applying any one of the compositions of the present invention to skin. The uneven skin tone can present itself as discolored skin. The composition can be applied to discolored skin (e.g., facial skin, arm skin, leg skin, scalp, neck skin, chess skin, abdomen skin, hand skin, etc.) selectively or to a greater skin area comprising the discolored skin. The discolored skin can be an age spot, blotchy skin, a freckle, hyperpigmented skin, skin suffering from melasma, skin that has been over-exposed to sun, etc. The method can also be used to prevent the appearance of uneven skin tone by topical application of the compositions disclosed throughout this specification to skin that is at risk of developing uneven skin tone. Skin at risk of developing uneven skin tone includes skin that has been over-exposed to sun, skin of pregnant women, people having or at risk of developing melasma, post-inflammatory hyperpigmentation (e.g., darkening of skin after injury to skin such as an acne lesion or a burn). The compositions of the present invention can also be used to lighten skin by topically applying to skin (areas) that the user desires to lighten a composition disclosed in this specification.

The cosmetic compositions according to the invention are preferably intended for topical application, which is to be understood as the external application to the skin, in particular the face.

The (total) amount of the at least one compound of formula (I) in the cosmetic compositions according to the present inventin is preferably selected in the range of 0.5 ppm to 5'000 ppm, preferably in the 2.5 ppm to 250 ppm, most preferably in the range of 25ppm to 100 ppm, based on the total weight of the composition.

The term 'an effective amount' refers to an amount necessary to obtain the physiological effect. The physiological effect may be achieved by application of one dose or by repeated applications. The dosage administered may, of course, vary depending upon known factors, such as the physiological characteristics of the particular composition comprising the at least one compound of formula (I) and its mode and route of administration; the age, health and weight of the recipient; the nature and extent of the symptoms; the kind of concurrent treatment; the frequency of treatment; and the effect desired; and can be adjusted by a person skilled in the art. Preferably, the amount of the cosmetic composition to be applied to the skin is selected in the range of 0.1 to 3 mg/ cm² skin, such as preferably in the range of 0.1 to 2 mg/ cm² skin and most preferably in the range of 0.5 to 2 mg / cm² skin. In a preferred embodiment, the cosmetic composition is applied once or twice daily. In preferred embodiments of the present invention, the cosmetic composition is applied once or twice per day for a period of at least 10 days, even more preferably for a period of at least 20 days, such as most preferably for a period of at least 25 days, such as e.g. for a period of at least 28 days.

In a preferred embodiment of the present invention, the cosmetic composition is applied to the face. Particularly suitable application areas in the face include the forehead, the cheek under the eyes (dark circles under the eye), the cheek and the jawline. Most preferred is the area of the cheeks under the eyes as there the effect was particularly pronounced.

The term 'C₁-C₆alkyl' as used herein refers to unbranched C₁-C₆alkyl or branched C₃-C₆alkyl groups such as methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl groups.

The term 'arC₁-C₆alkyl' as used herein refers to a -C₁-C₆alkyl-aryl wherein the term 'aryl' is e.g. a phenyl, indanyl or naphthyl group.

The term 'heteroarylC₁-C₆alkyl' as used herein refers to a -C₁-C₆alkyl-heteroaryl wherein the term "heteroaryl" refers to a 5- or 6-membered aromatic ring containing one or more heteroatoms, viz., N, O or S; these heteroaromatic rings may be fused to other aromatic systems.

Examples for the 5- to 7-membered, saturated ring, that R¹ and R² or R¹ and R⁵, respectively, may form together with the residue to which they are bound, are pyrrolidinyl, piperidinyl, piperazinyl, morpholinyl, azepinyl, oxazolidinyl, thiazolidinyl and 1,2,3,4-tetrahydroquinolinyl.

It is well understood, that the present invention encompasses the compounds of formula (I) as optically pure isomers such as e.g. as pure enantiomers or stereoisomers as well as mixtures of different isomers such as e.g. as racemates, or mixtures of diastereoisomers. It is well understood, that the term «cosmetically acceptable salt" refers to non-toxic acid addition salts, i.e. salts that are physiologically acceptable and may be used in contact with skin, lips, hair or gums without causing any undesirable or deleterious effects. Preferably, the term 'or a cosmetically acceptable salt thereof' refers to compounds of formula (I) in the form of an acid addition salt such as in the form of a chloride, an acetate, a me-sylate or a trifluoroacetate salt. Alternatively, the salt may be formed by reaction with an alkali or earth alkaline base resulting in the respective alkali or earth alkaline salt such as in particular the respective lithium, sodium, potassium, magnesium or calcium salts.

Most preferred, in all embodiments of the present invention, are the compounds of formula (I) in the form of their acetates or trifluoroacetates. Such salts are easily prepared by a person skilled in the art.

In all embodiments of the present invention R¹, R⁴ and R⁵ are preferably H.

In all embodiments of the present invention R² is preferably H and methyl, most preferably H.

In all embodiments of the present invention R³ is preferably arC₁-C₆alkyl, most preferably benzyl.

In all embodiments of the present invention n is preferably 0 or 1, most preferably 1.

Most preferred in all embodiments of the present invention is a compound of formula (I), wherein R¹, R², R⁴ and R⁵ are H, R³ is benzyl and n is 1.

The compounds of formula (I) can be prepared as e.g. disclosed in US 2009/0111731.

Particular advantageous in all embodiments of the present invention is the dipeptide having the sequence H-(beta-Ala)-Pro-Dab-NH-benzyl, in particular as diacetate. This compound is also known as Dipeptide Diaminobutyroyl Benzylamide Diacetate (INCI) [CAS 823202-99-9], and is commercially available as SYN^{®}-AKE from DSM Nutritional products Ltd.

The term 'cosmetic composition' as used herein refers to compositions which are used to treat, care for or improve the appearance of the skin and/or the scalp. Particular advantageous cosmetic compositions are skin care preparations.

As the compositions according to the invention are intended for topical application, they comprise a physiologically acceptable medium, i.e. a medium compatible with the skin. In particular, the physiologically acceptable medium is a cosmetically acceptable carrier.

The term 'cosmetically acceptable carrier' as used herein refers to a physiologically acceptable medium which is compatible with keratinous substances such as in particular the skin. Suitable carriers are well known in the art and are selected based on the end-use application. Preferably, the carriers of the present invention are suitable for application to skin (e.g., sunscreens, creams, milks, lotions, masks, serums, hydrodispersions, foundations, creams, creamgels, or gels etc.). Such carriers are well-known to one of ordinary skill in the art and can include one or more compatible liquid or solid filler diluent, excipient, additive or vehicle which are suitable for application to skin. The exact amount of carrier will depend upon the level of the compound of formula (I) and any other optional ingredients that one of ordinary skill in the art would classify as distinct from the carrier (e.g., other active components). The compositions of the present invention preferably comprise from about 75% to about 99.999%, more preferably from about 85% to about 99.99%, still more preferably from 90% to about 99%, and most preferably, from about 93% to about 98%, by weight of the composition, of a carrier.

The cosmetic compositions of the present invention can be formulated into a wide variety of product types, including creams, waxes, pastes, lotions, milks, mousses, gels, oils, tonics, and sprays. Preferably the compounds of formula (I) are formulated into lotions, creams, gels, and tonics. These product forms may be used for a number of applications, including, but not limited to, hand and body lotions, facial moisturizers, anti-ageing preparations, make-ups including foundations, and the like. Any additional components required to formulate such products vary with product type and can be routinely chosen by one skilled in the art.

If compositions of the present invention are formulated as an aerosol and applied to the skin as a spray-on product, a propellant is added to the composition.

The cosmetic compositions according to the present invention can be prepared by conventional methods in the art such as e.g. by admixing a compound of formula (I) with all the definitions and preferences given herein with the cosmetically acceptable carrier.

The cosmetic compositions of the invention (including the carrier) may comprise further conventional cosmetic adjuvants and additives, such as preservatives/antioxidants, fatty substances/oils, water, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the cosmetic compositions according to the invention may also comprise further cosmetically active ingredients conventionally used in cosmetic compositions. Exemplary active ingredients encompass skin lightening agents; UV-filters, agents for the treatment of hyperpigmentation; agents for the prevention or reduction of inflammation; firming, moisturizing, soothing, and/ or energizing agents as well as agents to improve elasticity and skin barrier.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the cosmetic excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The cosmetic compositions according to the present invention may be in the form of a suspension or dispersion in solvents or fatty substances, or alternatively in the form of an emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. oil-in-water-in oil (O/W/O) or water-in-oil-in-water (W/O/W) type), pickering emulsion, hydrogel, alcoholic gel, lipogel, one- or multiphase solution or vesicular dispersion or other usual forms, which can also be applied by pens, as masks or as sprays.

If the cosmetic composition is an emulsion, such as in particular an O/W, W/O, Si/W, W/Si, O/W/O, W/O/W multiple or a pickering emulsion, then the amount of the oily phase present in such cosmetic emulsions is preferably at least 10 wt.-%, such as in the range of 10 to 60 wt.-%, preferably in the range of 15 to 50 wt.-%, most preferably in the range of 15 to 40 wt.-%, based on the total weight of the cosmetic composition.

In one embodiment, the cosmetic compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the cosmetic composition according to the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol^{®} A from DSM Nutritional Products Ltd.), diethanolamine cetyl phosphate (e.g. as Amphisol^{®} DEA from DSM Nutritional Products Ltd.), potassium cetyl phosphate (e.g. as Amphisol^{®} K from DSM Nutritional Products Ltd.), sodium cetearylsulfate, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the cosmetic composition.

Particular suitable O/W emulsifiers to be used in the cosmetic compositions according to the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate.

Particular suitable O/W emulsifiers to be used in the cosmetic compositions according to the invention are the cetyl phosphates such as preferably potassium cetyl phosphate which is e.g. commercially available as Amphisol^{®} K at DSM Nutritional Products Ltd Kai-seraugst.

Another particular suitable class of O/W emulsifiers are non-ionic self-emulsifying systems derived from olive oil e.g. known as (INCI Name) cetearyl olivate and sorbitan olivate (chemical composition: sorbitan ester and cetearyl ester of olive oil fatty acids) sold under the tradename OLIVEM 1000.

In one particular embodiment, the invention relates to cosmetic compositions with all the definitions and preferences given herein in the form of O/W emulsions comprising an oily phase dispersed in an aqueous phase in the presence of an O/W emulsifier wherein the O/W emulsifier is potassium cetyl phosphate. The amount of oily phase in such O/W emulsions is preferably at least 10 wt.-%, more preferably in the range of 10 to 60 wt.-%, most preferably in the range of 15 to 50 wt.-%, such as in the range of 15 to 40 wt.-%.

The cosmetic compositions according to the invention in general have a pH in the range of 3 to 10, preferably a pH in the range of 4 to 8 and most preferably a pH in the range of 4 to 7.5. The pH can easily be adjusted as desired with suitable acids, such as e.g. citric acid, or bases, such as sodium hydroxide (e.g. as aqueous solution), triethanolamine (TEA Care), Tromethamine (Trizma Base) and Aminomethyl Propanol (AMP-Ultra PC 2000), according to standard methods in the art.

The following examples are provided to further illustrate the compositions and effects of the present invention. These examples are illustrative only and are not intended to limit the scope of the invention in any way.

### Experimental part

### 1. Material & Methods

This was a placebo controlled, full face, and parallel groups study. Volunteers had to apply the cosmetic formulations twice daily for 29 days. 54 female Asian volunteers participated in the study divided into Placebo group and Active group (formulation containing 4% SYN^{®}-AKE).

The volunteers were photographed with cross polarized lighting of a Visia CR^{®} device (Canfield, Parsippany, US) at 45° angles from left and right. L*a*b* values were calculated directly on images using a defined algorithm. Differences were computed and significances calculated using Studen's t-test (if values followed a normal distribution) or Wilcoxon Signed Rank test (if value did not follow a normal distribution).

### 2. Clinical Study

A double blind parallel group study was performed with Asian female volunteers aged 41-55. 27 volunteers (mean age 47years) received a placebo formulation and 27 volunteers (mean age 48 years) received the active formulation containing SYN^{®}-AKE. The placebo respectively active formulation was applied twice daily for 28 days to face. At the end of the study pictures were taken for computational analysis of skin toneas outlined above.

**Table 1: PlacebolActive formulation**

| **Ingredients** | **INCI Name** | **Placebo** | **Active** |
|---|---|---|---|
| | | [%] | |
| AMPHISOL^{®} K | POTASSIUM CETYL PHOSPHATE | 1.00 | 1.00 |
| Ecorol 16/98P | CETYL ALCOHOL | 3.00 | 3.00 |
| Cutina CP | CETYL PALMITATE | 1.50 | 1.50 |
| Eutanol G | OCTYLDODECANOL | 3.00 | 3.00 |
| Pemulen TR-1 | ACRYLATES/C10-30 ALKYL ACRYLATE CROSSPOLYMER | 0.10 | 0.10 |
| 1,3-Butylenglycol | BUTYLENE GLYCOL | 3.00 | 3.00 |
| WATER DEM. | AQUA | Ad 100 | Ad 100 |
| Dow Corning 345 Fluid | CYCLOPENTASILOXANE, CYCLOHEXASILOXANE | 2.50 | 2.50 |
| Natriumhydroxid 30% | AQUA, SODIUM HYDROXIDE | 0.09 | 0.09 |
| SYN^{®}-AKE | Dipeptide Diaminobutyroyl Benzylamide Diacetate, Glycerin, Aqua | 0 | 4.00 |
| Euxyl PE 9010 | PHENOXYETHANOL, ETHYLHEX-YLGLYCERIN | 1.00 | 1.00 |
| Frag 49424902 Chloe | Perfume | 0.05 | 0.05 |

### 3. Results

| **Facial area** | | **L* value difference: day 29 to day 0** | | **Significance (p-value)** | | **a* value difference: day 29 to day 0** | | **Significance (p-value)** | |
|---|---|---|---|---|---|---|---|---|---|
| **Forehead** | | | | | | | | | |
| Placebo | | -0.05 | | 0.705 | | -0.06 | | 0.443 | |
| Invention | | **0.36** | | **0.064** | | **-0.24** | | **0.040** | |
| **Cheek under eyes (dark circles)** | | | | | | | | | |
| Placebo | | -0.13 | | 0.744 | | -0.09 | | 0.082 | |
| Active | | **0.36** | | **0.016** | | **-0.35** | | **0.018** | |
| **Cheek** | | | | | | | | | |
| Placebo | | 0.10 | | 0.185 | | -0.12 | | 0.071 | |
| Active | | **0.29** | | **0.081** | | **-0.26** | | **0.057** | |
| **Jawline** | | | | | | | | | |
| Placebo | | 0.07 | | 0.502 | | -0.13 | | 0.360 | |
| Active | | 0.09 | | 0.671 | | **-0.29** | | **0.026** | |

As can be retrieved from the results a significant improvement in the skin tone was observed versus the Placebo formulation not containing the active.

## Claims

1. Use of a compound of formula (I). 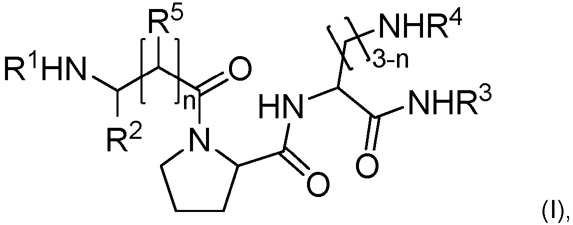 wherein
n represents 0, 1 or 2,
R¹ and R⁴ - independently of each other - are selected from the group consisting of H, C₁-C₆-alkyl, amidino or tetra-C₁-C₆-alkylamidinium,
R² is H or C₁-C₆-alkyl, or R¹ and R² together with the residue to which they are bound form a 5- to 7-membered, saturated ring,
R³ is selected from the group consisting of C₁-C₆alkyl, arC₁-C₆alkyl and heteroaryl-C₁-C₆alkyl, and
R⁵ is H or, when n is 1, also NH₂, or R⁵ and R¹ together with the residue to which they are bound form a 5- to 7-membered, saturated ring,
or a cosmetically acceptable salt thereof for evening out skin tone, for brightening of skin and/ or for reducing dark circles under the eye area.

2. The use according to claim 1, **characterized in that** the L* value of the skin is increased and the a* value is decreased compared to non-treated skin.

3. The use according to claim 1 or 2, **characterized in that** R¹, R⁴ and R⁵ are H.

4. The use according to any one of claims 1 to 3, **characterized in that** R³ is arC₁-C₆alkyl, preferably benzyl.

5. The use according to any one of claims 1 to 3, **characterized in that** n is 0 or 1, preferably 1.

6. The use according to claim 1 or 2, **characterized in that** R¹, R², R⁴ and R⁵ are H, R³ is benzyl and n is 1.

7. The use according to any one of claims 1 to 6, **characterized in that** the compound of formula (I) is H-(beta-Ala)-Pro-Dab-NH-benzyl, in particular in the form of its diacetate.

8. The use according to any one of claims 1 to 7, **characterized in that** the compound of formula (I) is used incorporated in a topical administered cosmetic composition comprising the compound of formula (I) in an amount selected in the range of 0.5 ppm to 5'000 ppm, preferably in the range of 2.5 ppm to 250 ppm, most preferably in the range of 25 ppm to 100 ppm, based on the total weight of the composition.

9. The use according to claim 8, **characterized in that** the amount of the cosmetic composition to be applied to the skin is selected in the range of 0.1 to 3 mg/ cm² skin, preferably in the range of 0.1 to 2 mg/ cm² skin, most preferably in the range of 0.5 to 2 mg / cm² skin.

10. The use according to claim 8 or 9, **characterized in that** the cosmetic composition is applied once or twice daily.

11. The use according to any one of claims 8 to 10, **characterized in that** the cosmetic composition is a skin care preparation.

12. The use according to any one of claims 8 to 11, **characterized in that** the composition is an O/W emulsion comprising an oily phase dispersed in an aqueous phase in the presence of an O/W emulsifier.

13. The use according to claim 12, **characterized in that** the O/W emulsifier is a cetyl phosphate.

14. A compound of formula (I) 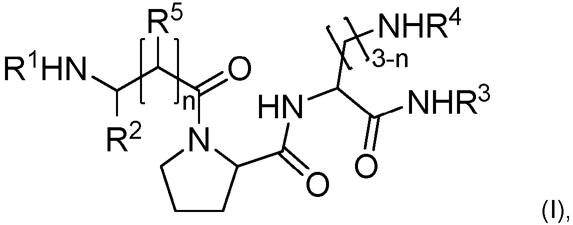 wherein
n represents 0, 1 or 2,
R¹ and R⁴ - independently of each other - are selected from the group consisting of H, C₁-C₆alkyl, amidino or tetra-C₁-C₆-alkylamidinium,
R² is H or C₁-C₆-alkyl, or R¹ and R² together with the residue to which they are bound form a 5- to 7-membered, saturated ring,
R³ is selected from the group consisting of C₁-C₆alkyl, arC₁-C₆alkyl and het-eroarylC₁-C₆alkyl, and
R⁵ is H or, when n is 1, also NH₂, or R⁵ and R¹ together with the residue to which they are bound form a 5- to 7-membered, saturated ring,
or a cosmetically acceptable salt thereof for use in the treatment of reducing inflammation-related discoloration, erythema and/ or pigmentation of skin.

## Patentansprüche

1. Verwendung einer Verbindung der Formel (I) 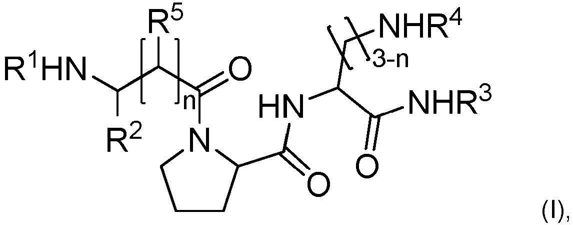 wobei
n für 0, 1 oder 2 steht,
R¹ und R⁴ - unabhängig voneinander - aus der Gruppe bestehend aus H, C₁-C₆-Alkyl, Amidino oder Tetra-C₁-C₆-alkylamidinium ausgewählt sind,
R² für H oder C₁-C₆-Alkyl steht oder R¹ und R² zusammen mit dem Rest, an den sie gebunden sind, einen 5- bis 7-gliedrigen, gesättigten Ring bilden,
R³ aus der Gruppe bestehend aus C₁-C₆-Alkyl, Ar-C₁-C₆-alkyl und Heteroaryl-C₁-C₆-alkyl ausgewählt ist und
R⁵ für H steht oder dann, wenn n für 1 steht, auch für NH₂ steht oder R⁵ und R¹ zusammen mit dem Rest, an den sie gebunden sind, einen 5- bis 7-gliedrigen, gesättigten Ring bilden,
oder eines kosmetisch unbedenklichen Salzes davon zum Ausgleichen des Hauttons, zum Aufhellen der Haut und/oder zum Verringern von dunklen Ringen unter dem Augenbereich.

2. Verwendung nach Anspruch 1, **dadurch gekennzeichnet, dass** im Vergleich zu unbehandelter Haut der L*-Wert der Haut erhöht und der a*-Wert erniedrigt wird.

3. Verwendung nach Anspruch 1 oder 2, **dadurch gekennzeichnet, dass** R¹, R⁴ und R⁵ für H stehen.

4. Verwendung nach einem der Ansprüche 1 bis 3, **dadurch gekennzeichnet, dass** R³ für Ar-C₁-C₆-alkyl, vorzugsweise Benzyl, steht.

5. Verwendung nach einem der Ansprüche 1 bis 3, **dadurch gekennzeichnet, dass** n für 0 oder 1, vorzugsweise 1, steht.

6. Verwendung nach Anspruch 1 oder 2, **dadurch gekennzeichnet, dass** R¹, R², R⁴ und R⁵ für H stehen, R³ für Benzyl steht und n für 1 steht.

7. Verwendung nach einem der Ansprüche 1 bis 6, **dadurch gekennzeichnet, dass** es sich bei der Verbindung der Formel (I) um H-(beta-Ala)-Pro-DAB-NH-Benzyl, insbesondere in Form ihres Diacetats, handelt.

8. Verwendung nach einem der Ansprüche 1 bis 7, **dadurch gekennzeichnet, dass** die Verbindung der Formel (I) in einer topisch verabreichten kosmetischen Zusammensetzung verwendet wird, welche die Verbindung der Formel (I) in einer Menge umfasst, die im Bereich von 0,5 ppm bis 5000 ppm, vorzugsweise im Bereich von 2,5 ppm bis 250 ppm, ganz besonders bevorzugt im Bereich von 25 ppm bis 100 ppm, bezogen auf das Gesamtgewicht der Zusammensetzung, ausgewählt ist.

9. Verwendung nach Anspruch 8, **dadurch gekennzeichnet, dass** die Menge der kosmetischen Zusammensetzung, die auf die Haut aufzubringen ist, im Bereich von 0,1 bis 3 mg/cm² Haut, vorzugsweise im Bereich von 0,1 bis 2 mg/cm² Haut, ganz besonders bevorzugt im Bereich von 0,5 bis 2 mg/cm² Haut, gewählt wird.

10. Verwendung nach Anspruch 8 oder 9, **dadurch gekennzeichnet, dass** die kosmetische Zusammensetzung ein- oder zweimal täglich aufgebracht wird.

11. Verwendung nach einem der Ansprüche 8 bis 10, **dadurch gekennzeichnet, dass** es sich bei der kosmetischen Zusammensetzung um eine Hautpflegezubereitung handelt.

12. Verwendung nach einem der Ansprüche 8 bis 11, **dadurch gekennzeichnet, dass** es sich bei der Zusammensetzung um eine O/W-Emulsion handelt, die eine in Gegenwart eines O/W-Emulgators in einer wässrigen Phase dispergierte ölige Phase umfasst.

13. Verwendung nach Anspruch 12, **dadurch gekennzeichnet, dass** es sich bei dem O/W-Emulgator um ein Cetylphosphat handelt.

14. Verbindung der Formel (I) 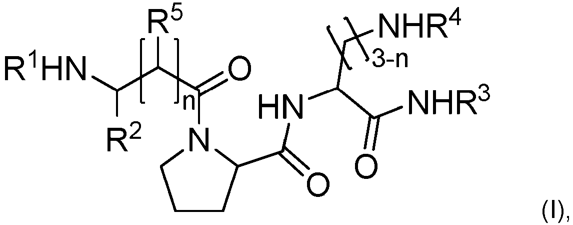 wobei
n für 0, 1 oder 2 steht,
R¹ und R⁴ - unabhängig voneinander - aus der Gruppe bestehend aus H, C₁-C₆-Alkyl, Amidino oder Tetra-C₁-C₆-alkylamidinium ausgewählt sind,
R² für H oder C₁-C₆-Alkyl steht oder R¹ und R² zusammen mit dem Rest, an den sie gebunden sind, einen 5- bis 7-gliedrigen, gesättigten Ring bilden,
R³ aus der Gruppe bestehend aus C₁-C₆-Alkyl, Ar-C₁-C₆-alkyl und Heteroaryl-C₁-C₆-alkyl ausgewählt ist und
R⁵ für H steht oder dann, wenn n für 1 steht, auch für NH₂ steht oder R⁵ und R¹ zusammen mit dem Rest, an den sie gebunden sind, einen 5- bis 7-gliedrigen, gesättigten Ring bilden,
oder ein kosmetisch unbedenkliches Salz davon zur Verwendung bei der Behandlung zur Verringerung von entzündungsbedingter Verfärbung, Erythem und/oder Pigmentierung der Haut.

## Revendications

1. Utilisation d'un composé de formule (I), 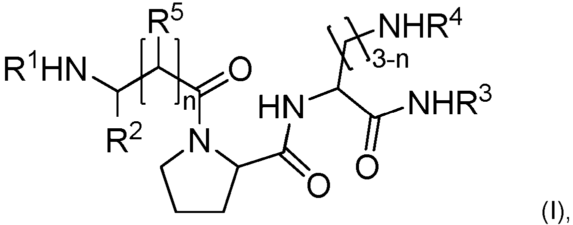 dans laquelle
n représente 0, 1 ou 2,
R¹ et R⁴ - indépendamment l'un de l'autre - sont choisis dans le groupe constitué par H, alkyle en C₁-C₆, amidino ou tétra-C₁-C₆-alkylamidinium,
R² est H ou alkyle en C₁-C₆, ou R¹ et R², conjointement avec le radical auquel ils sont liés, forment un cycle saturé à 5 à 7 chaînons,
R³ est choisi dans le groupe constitué par C₁-C₆alkyle, arC₁-C₆alkyle et hétéro-aryl-C₁-C₆alkyle, et
R⁵ est H ou, lorsque n est 1, également NH₂, ou R⁵ et R¹, conjointement avec le radical auquel ils sont liés, forment un cycle saturé à 5 à 7 chaînons,
ou d'un sel acceptable sur le plan cosmétique correspondant pour uniformiser la carnation, pour éclaircir la peau et/pour atténuer les cernes sous la zone oculaire.

2. Utilisation selon la revendication 1, **caractérisée en ce que** la valeur L* de la peau est augmentée et la valeur a* est diminuée par rapport à la peau non traitée.

3. Utilisation selon la revendication 1 ou 2, **caractérisée en ce que** R¹, R⁴ et R⁵ sont H.

4. Utilisation selon l'une quelconque des revendications 1 à 3, **caractérisée en ce que** R³ est arC₁-C₆alkyle, de préférence benzyle.

5. Utilisation selon l'une quelconque des revendications 1 à 3, **caractérisée en ce que** n est 0 ou 1, de préférence 1.

6. Utilisation selon la revendication 1 ou 2, **caractérisée en ce que** R¹, R², R⁴ et R⁵ sont H, R³ est benzyle et n est 1.

7. Utilisation selon l'une quelconque des revendications 1 à 6, **caractérisée en ce que** le composé de formule (I) est H-(béta-Ala)-Pro-Dab-NH-benzyle, en particulier sous la forme de son diacétate.

8. Utilisation selon l'une quelconque des revendications 1 à 7, **caractérisée en ce que** le composé de formule (I) est utilisé incorporé dans une composition cosmétique administrée par voie topique comprenant le composé de formule (I) en une quantité choisie dans la plage de 0,5 ppm à 5 000 ppm, de préférence dans la plage de 2,5 ppm à 250 ppm, plus préférablement dans la plage de 25 ppm à 100 ppm, par rapport au poids total de la composition.

9. Utilisation selon la revendication 8, **caractérisée en ce que** la quantité de la composition cosmétique à appliquer sur la peau est choisie dans la plage de 0,1 à 3 mg/cm² de peau, de préférence dans la plage de 0,1 à 2 mg/cm² de peau, plus préférablement dans la plage de 0,5 à 2 mg/cm² de peau.

10. Utilisation selon la revendication 8 ou 9, **caractérisée en ce que** la composition cosmétique est appliquée une ou deux fois par jour.

11. Utilisation selon l'une quelconque des revendications 8 à 10, **caractérisée en ce que** la composition cosmétique est une préparation de soin de la peau.

12. Utilisation selon l'une quelconque des revendications 8 à 11, **caractérisée en ce que** la composition est une émulsion H/E comprenant une phase huileuse dispersée dans une phase aqueuse en la présence d'un émulsifiant H/E.

13. Utilisation selon la revendication 12, **caractérisée en ce que** l'émulsifiant H/E est un phosphate de cétyle.

14. Composé de formule (I), 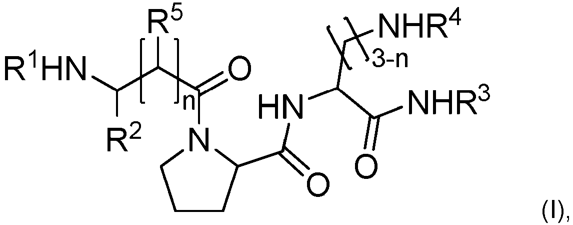 dans laquelle
n représente 0, 1 ou 2,
R¹ et R⁴ - indépendamment l'un de l'autre - sont choisis dans le groupe constitué par H, C₁-C₆alkyle, amidino ou tétra-C₁-C₆-alkylamidinium,
R² est H ou alkyle en C₁-C₆, ou R¹ et R², conjointement avec le radical auquel ils sont liés, forment un cycle saturé à 5 à 7 chaînons,
R³ est choisi dans le groupe constitué par C₁-C₆alkyle, arC₁-C₆alkyle et hétéro-aryl-C₁-C₆alkyle, et
R⁵ est H ou, lorsque n est 1, également NH₂, ou R⁵ et R¹, conjointement avec le radical auquel ils sont liés, forment un cycle saturé à 5 à 7 chaînons,
ou sel acceptable sur le plan cosmétique correspondant pour une utilisation dans le traitement de la réduction d'une décoloration liée à une inflammation, d'un érythème et/ou de la pigmentation de la peau.
